# EUROPEAN PATENT SPECIFICATION

(11) **EP 1 009 284 B1**
(45) Date of publication and mention of the grant of the patent: **28.01.2004**
(21) Application number: 98945887.2
(22) Date of filing: 03.09.1998
(51) Int. Cl.: A61B 6/04

(54) **RADIOLUCENT TABLE EXTENSION ASSEMBLY**
RÖNTGENSTRAHLDURCHLÄSSIGE VERLÄNGERUNGSTISCHANORDNUNG
ENSEMBLE EXTENSION DE TABLE PERMEABLE AUX RAYONS X

(30) Priority: 03.09.1997 US 922969; 24.04.1998 US 83014 P
(43) Date of publication of application: 21.06.2000
(73) Proprietor: OHIO MEDICAL INSTRUMENT COMPANY, INC., Cincinnati, OH 45227 (US)
(72) Inventor: DINKLER, Charles, E., c/o Ohio Med.Instr.Comp.Inc., Cincinnati, OH 45227 (US); KANTROWITZ, Allen, c/o Ohio Med.Instr.Comp.Inc., Cincinnati, OH 45227 (US); MUN, In, Ki, Miami Beach, FL 33140 (US)
(74) Representative: Schmidt, Steffen J., Dipl.-Ing.
(86) International application number: PCT/US1998/018405
(87) International publication number: WO 1999/011176

(56) References cited:
- EP-A- 0 104 591
- WO-A-95/21597
- US-A- 5 276 927
- US-A- 5 427 436

## Description

### Field of the Invention

This invention relates to beds and more particularly, to an improved surgical operating table.

### Background of the Invention

With current medical practices, it is common for a patient to undergo a diagnostic scanning procedure, which is normally performed in a separate suite containing the scanning machine and dedicated to scanning procedures. The scanning machine may be a CT, MRI, or other scanning device. Thereafter, the scan data is utilized in a surgical planning process, which conventionally takes place at a location, for example, an office or an operating room. In some surgical procedures, the scanning data is utilized with a system for post processing the scan data acquired during imaging. Further, the imaging system may be located in a surgical suite, and the surgical planning performed before and during surgical procedure utilizing the imaging system and scan data.

During the scanning procedure, the patient must maintain a perfectly still and motionless posture, and while most often, the patient simply lies on a scanning support table, in some situations, the patient may be supported in the desired scanning position with pads, straps or other supports. Further, the support on which the patient rests is normally radiolucent, that is, transparent to the scanning device, so that the support does not compromise the utility of the scanned image. Further, the patient support used for scanning normally translates with respect to the imaging device. Translation of the patient support permits the patient to be moved into the scanning field or zone of the scanning machine.

After the scanning process is completed, often the patient is then moved to an operating room which requires either that the patient walk, or be carried, for example, by transferring the patient from the scanning table to an operating table. Alternatively, as illustrated in U.S. Patent No. 5,475,884, the patient may be supported on a portable support plate, which is easily moved between the scanning table and the operating table. The scan data is often used in a post processing imaging system for surgical planning purposes both prior to and during surgery. If during or after a surgical process, it is desired to scan a patient again, the patient must be moved from the operating room to the scanning suite, transferred to and from the operating table to the scanning table, and after scanning, transferred back to the operating table and returned to the operating room. The above process is cumbersome, time consuming and potentially risky for the patient.

Some newer scanning machines are substantially reduced in size. One such machine is shown in Figs. 2 and 3 of U.S. Patent No. 5,499,415, which show an annular-shaped scanner mounted on a wheel-supported frame, to enable the scanner to be used at multiple sites. Consequently, such scanning machines do not require their own suite or room, but instead, they may be used within the operating suite itself. Thus, in an operating room, the patient may be scanned; the surgical planning performed; an operative procedure executed; and the patient scanned again to determine the current status of the operative procedure. Based on the new scanned images, the operative procedure can be continued and the above process repeated as necessary.

A limitation of the current state-of-the-art is that the posture of the patient during the scanning process is often different from the patient's posture during surgery. If a patient is positioned in one posture on a scanning table during the scanning process, and then is moved to an operating table, that motion of the patient may cause the position of the target to change with respect to the body surface. During surgery, this problem is compounded by tissue shifts attendant to the opening of body cavities, removal of body fluid or tissues and tissue retractions. Thus, while such motion may be small, any motion of the target will reduce or compromise the utility of the preoperative scan data.

The solution to these problems is to scan the patient in the operating room during surgery while the patient is maintained in the surgical posture.

While current scanning tables are radiolucent and provide a translation to move the patient into the scanning machine, such scanning tables do not have the accessories required to attach, support and stabilize surgical instrumentation and to properly support the patient's body in the desired surgical posture. Further, while operating tables contain numerous accessories and couplings to which surgical instrumentation may be attached and supported, most operating tables are not compatible with scanning instrumentation. Thus, as presently known, scanning tables cannot be used as operating tables, and generally, operating tables are inappropriate for use as scanning tables.

WO 95/21597 describes a patient trauma table for use in combination with a medical imaging system or scanner having an enclosed scanning zone. The table comprises a radiolucent elongated patient palette or panel attached to the table for suspending a patient so that he or she is cantilevered relative to a base member of the table. The table and the radiolucent panel are movable relative to the scanner by wheels supporting the base member. Thus, the panel can be located within the scanning zone. WO 95/21597 does not describe any possibility to properly support the patient's body in a desired posture.

US-A-5,276,927 shows a head support for a patient support table which is used to position a patient's head during the taking of an X-ray. The head support comprises skull securing means being mounted to supporting means. The skull securing means and the supporting means are made substantially from radiolucent material. The only described embodiment of a skull securing means shows a clamp securing the patient's head in a desired position. The patient's head rests with its back on a middle portion of the clamp. The clamp further comprises two lateral end portions at which two screws are located, respectively, which can be tightened against the patient's temples. Thus, the positioning of the patient's head is defined by three points only, i.e. the middle portion and the two lateral end portions of the clamp. The clamp itself is rigid. The three points for positioning the patient's head can not be moved relative to each other or adjusted to different.head sizes of different patients. Therefore, the field of application for such a clamp is limited to certain head sizes and a number of clamps varying in size have to be provided. Furthermore, the patient's head resting on the middle portion of the clamp is supported only by a rest having a very small support area. Resting on that point is painful for the patient. Thus, the skull securing means appears not to be applicable for a long period as it is necessary for a surgucal procedure which takes much more time than the taking of an X-ray.

US-A-5,427,436 describes a headrest which is used on operating tables. The headrest shows the form of a horse shoe.

It is an object of this invention to overcome the above-described limitations in the prior art, by facilitating the function of supporting a patient in a desired position in a manner which readily accommodates successive surgical or scanning procedures.

### Summary of the Invention

The present invention achieves the above-stated objective with a radiolucent table extension that connects to a surgical table and permits a patient to be positioned on the table in a posture suitable for successive surgical or scanning procedures, with the head and the upper torso of the patient supported on the table extension and the radiolucent table extension including additional cooperative components such as a radiolucent horseshoe headrest and a radiolucent skull clamp to positively hold the patient relative to extension. The radiolucent table extension is cantilevered from one end of the surgical table and it is shaped so that it may be moved in a relative manner into a toroidal shaped scanning zone of an upright annular scanning machine. This permits the patient to be scanned in the desired surgical posture. This radiolucent table extension is especially useful for those procedures in which it is desirable to maintain the patient in a desired position during successive scanning or surgical procedures.

By operatively connecting the toroidal scanner to an imaging system, so that the imaging system may store data representative of scans of the patient taken in the scanning zone, and by supporting the patient with the extension and fixing the position of the patient with the horseshoe headrest and the skull clamp, this invention facilitates the positioning of the patient during successive scans, thereby assuring the accuracy of the scanned data. This helps the surgeon to know almost immediately whether the surgical procedure accomplished its objective, or whether a subsequent surgical procedure may be necessary.

According to the principles of the present invention and in accordance with the preferred embodiments, a radiolucent table extension has a first end adapted to be attached to one end of a table. The table extension includes a contoured radiolucent member designed to support an upper torso and head of a patient with the rest of the patient's body being further supported by an adjacently located surface of the table. The member has a sufficiently narrow width to permit it to be extended, in cantilever fashion, into a scanning zone of portable CT scanning system. A tool support extends along a periphery of the member and is designed to receive and support at least one outboard stabilization device, such as a skull clamp, to positively hold the patient in a fixed position relative to the member. The member also includes means for locating at least one inboard stabilization device. More specifically, the inboard stabilization device inboard of the periphery, such as a radiolucent horseshoe headrest, also provides stabilized support for the patient. Therefore, the patient can be supported on the radiolucent table extension in the desired posture. The patient can then be conveniently scanned before a surgical procedure. After surgery, a subsequent scanning procedures may be performed if necessary or desired. Thus, the table extension has the advantage of not requiring that the patient be moved with respect to the table extension between successive scanning and surgical procedures.

Moreover, with updated scanned images readily available-for viewing via the imaging system, the surgeon can review the results of a surgical procedure to determine if a particular operation has been completely successful. For example, if the objective of the surgery was to completely remove a hematoma from the brain, a follow-up scan may enable the surgeon to use the imaging system to determine if the entire hematoma has been removed. If a subsequent scan shows that some of the "target" remains, then the surgeon can perform another surgical procedure, using the imaging system if desired, to achieve 100% removal of the target. Thus, this overall system facilitates successive scanning and surgical procedures, and the table extension assembly makes it possible to use this system more effectively, by assuring accurate and repeatable positioning of the patient.

In one aspect of the invention, a hinge mechanism mechanically couples the radiolucent member to an operating table, thereby permitting the table extension to be pivoted or rotated with respect to the table. Thus, the patient's head and upper torso may be raised or lowered and supported in any desired position to facilitate the scanning and operative procedures.

In a further aspect of the invention, the tool support extends along the periphery at one end of the table, but in another aspect of the invention, the tool support extends along the periphery including the lateral edges of the table. Thus, a wide variety of surgical instruments may be connected to the table extension to facilitate many different surgical procedures.

In still another aspect of the invention, the table extension includes a radiolucent horseshoe headrest removably mounted to the radiolucent member, inboard of the peripheral edge, and it is tiltable with respect to the member. This horseshoe headrest may surround a portion of an opening in the member, when viewed from above. The removable, tiltable horseshoe headrest optimizes versatility for the neurosurgeon in positioning a patient.

If desired, the tool support may be detachable from an external edge of the member. This enables a surgical drape, shaped in a bag-like form, to extend over the head of an intubated patient stabilized in position by the radiolucent horseshoe headrest. The headrest resides "inboard" of the peripheral edge with the patients' head and upper torso supported relative to the member.

Thereafter, the tool support can be connected to the edge of the member, with the drape sandwiched therebetween, and the skull clamp attached "outboard" to the tool support. This places the tooling connected to the tool support, i.e., the skull clamp, within the sterile field. This arrangement represents an advantageous option for some types of surgical and/or scanning procedures. For one thing, the drape is more closely confined to the table extension so that the surgeon and other hospital attendants can readily determine visually that the table extension and the patient can be extended within the scanning zone of the scanner without impediment. For some procedures, this arrangement also simplifies connection of the skull clamp.

These and other objects and advantages of the present invention will become more readily apparent during the following detailed description taken in conjunction with the drawings herein.

### Brief Description of the Drawings

Fig. 1 is a perspective view of a portion of an operating table including a radiolucent table extension assembly in accordance with the principles of the present invention.
Fig. 2 is a top plan view of the radiolucent table extension assembly of Fig. 1.
Fig. 3 is a side view in elevation of the radiolucent table extension assembly of Fig. 1.
Fig. 4 is a cross-section view taken along the line 4-4 of Fig. 2.
Fig. 5 is a cross-section of view taken along the line 5-5 of Fig. 2.
Fig. 6 is a top plan view of an alternative embodiment of the radiolucent table extension assembly in accordance with the principles of the present invention.
Fig. 7 is a perspective view of yet another alternative embodiment of a table extension assembly in accordance with the present invention.
Fig. 8 is a top plan view of the table extension assembly shown in Fig. 7, but with additional hardware shown, namely an inboard horseshoe headrest.
Fig. 9 is a side view of the table extension of Fig. 8, but also showing an outboard stabilization device, in this case a skull clamp, secured to the tooling support outboard of the edge of the table extension.
Fig. 10 is a side view, similar to Fig. 9, showing the inboard horseshoe headrest tilted relative to the table extension assembly.

### Detailed Description of the Invention

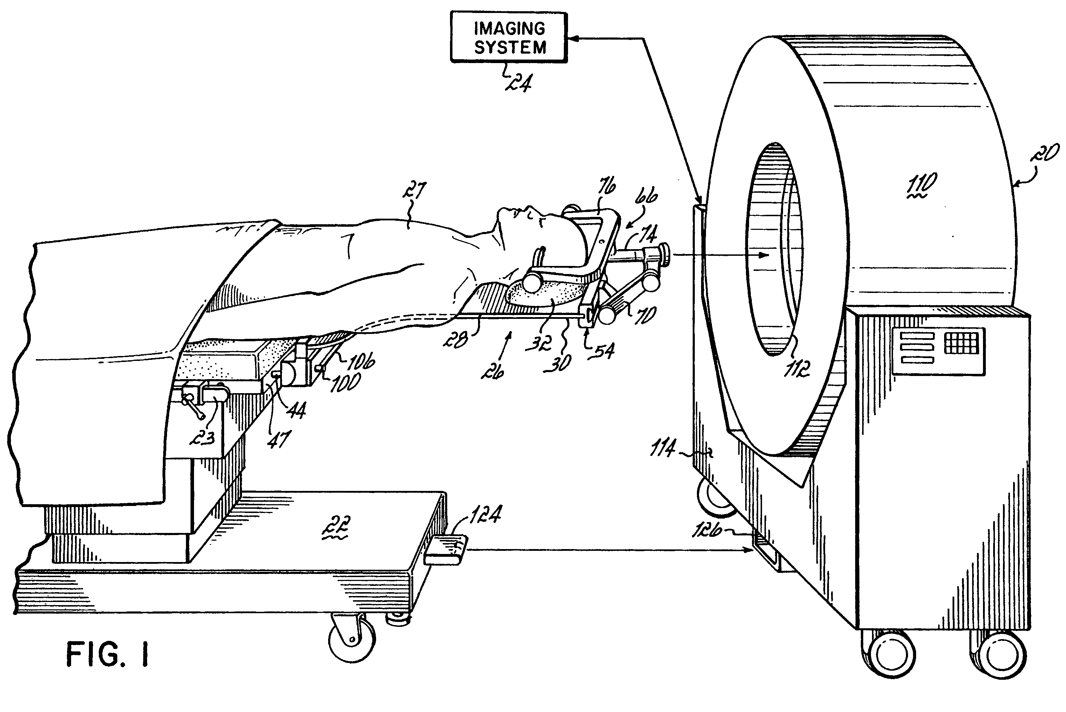

Referring to Fig. 1, a portable CT scanning system 20 is located in an operating suite with an operating table 22. The CT scanning system may be either a mobile system such as that commercially available from Analogic of Peabody, Massachusetts or a stationary scanning system such as that commercially available from General Electric Medical Systems of Milwaukee, Wisconsin. The operating table 22 may be one of many commercially available tables, for example, an operating table commercially available from Amsco of Erie, Pennsylvania, MDT Diagnostic Co. of N. Charleston, North Carolina, or other suppliers. The operating table has a lateral rail 23 extending along each side of the table to which retractors, clamps and other devices may be attached and stablely supported. A stereotactic image processing system 24 (cf.: US-5,695,501), for example, the MAYFIELD-ACCISS image processing system, commercially available from Ohio Medical Instrument Company, Inc. of Cincinnati, Ohio is operatively connected to the scanner 20 and responsive to scan data provided by the CT system 20, to provide selected images on a display screen of the scan data along selected planes. To facilitate the use of the operating table 22 with the CT system 20, one end of the operating table is used to support a radiolucent table extension, or table extension assembly, 26 in accordance with the present invention.

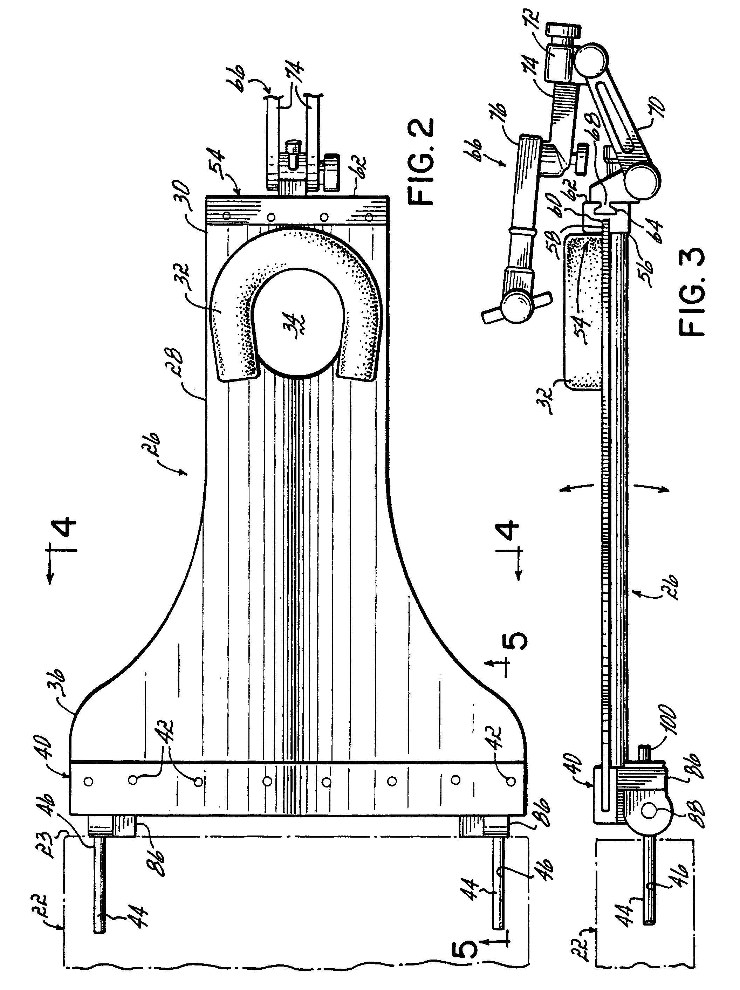

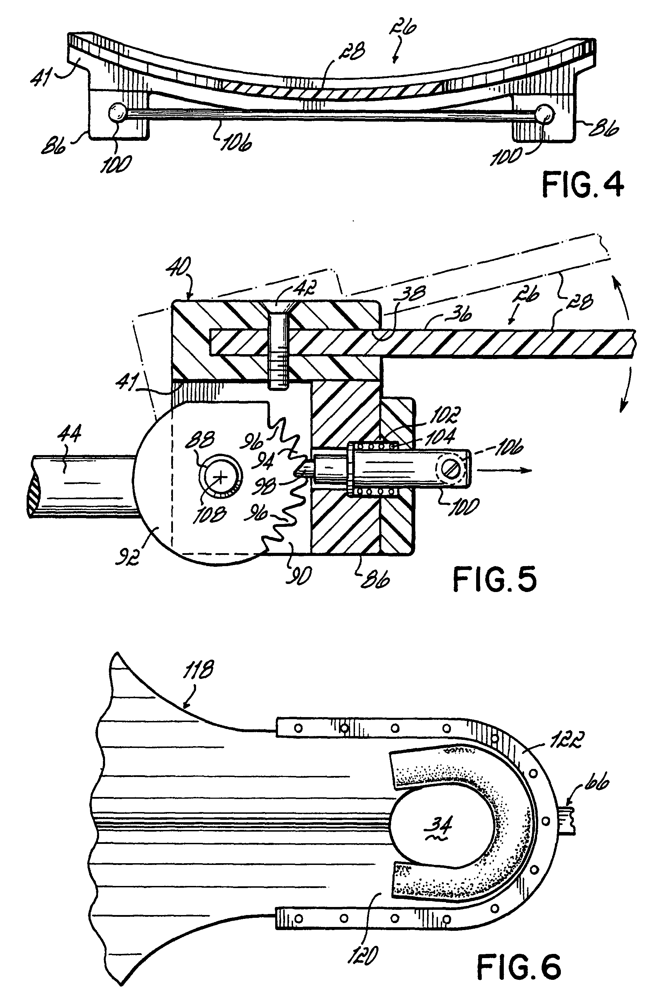

Referring to Fig. 2, the table extension 26 includes a support member or plate 28 made of radiolucent material, for example, wood, carbon graphite, etc, and the table extension 26 has a length to normally support the upper torso and head of a patient 27, the upper torso being defined as the portion of the patient's body above the waist including the head. As shown in Fig. 4, the patient support member 28 has a curved cross-sectional profile and has a laminated construction with a center layer of mahogany between two outer layers of carbon graphite, although the invention also contemplates molding the member 26 as one integral piece. The curve is normally a circular arc having a relatively large radius, for example, 28 inches, to generally conform to the shape of a patient. The support member 28 may have a length up to about 52 inches, although most procedures can be accommodated with a shorter-length, such as 36 inches. The outer or distal end 30 of the support member 28 includes a horseshoe headrest 32 that is generally U-shaped and filled with a gel to comfortably and properly support the patient's head. The headrest 32 surrounds an opening 34 within the support member 28. The opening 34 is sized to receive the face of a patient lying on the support member 28 in a prone position. The distal end 30 is narrower than the inner or fixed end 36, and the narrow profile of the distal end 20 of the support plate 28 facilitates positioning the distal end 30 in scanner 20 even if the table or the scanner 20 is tilted. The support member 28, when viewed from the top as shown in Fig. 2, has a profile that flares outward from the distal end 30 to the fixed end 36. The width of the support member 28 at the fixed end 36 is generally greater than the distance between the holes 46 and is normally equal to the width of the operating table 22.

Referring to Fig. 5, the support member 28 is secured at its fixed end 36 within a slot 38 of an attachment base 40. Fasteners, for example, screws 42 are used to clamp and secure the support plate 28 within the attachment base 40. The attachment base 40 is mechanically linked to support shafts 44, which extend longitudinally from the fixed end of the support base 40 and are sized to fit into holes 46 of the table 22. Thus, the support plate 28 provides an extension of and is cantilevered from the end 23 of the table 22.

Referring to Figs. 1-3, an instrument or tool support or rail 54 is attached to the periphery of the distal end 30 of the support plate 28. The tool support 54 may be made from a "DELRIN®" acetal polymer material, a polyethersuylfone ("PES") material or a carbon graphite. An inner directed side 56 of the tool support 54 includes a slot 58 for receiving the portion of the periphery 60 of the support plate 28. The support plate 28 may be secured in the slot 58 using fasteners or adhesives or both. The slot 58 is curved with respect to a radius sweeping a vertical plane that is generally perpendicular to and extends across the width of the support plate 28. An outer directed side 62 of the tool support 54 includes a second slot 64 that is generally parallel to a longitudinal center line of the tool support 54. Thus, when viewed from the end of the support plate 28, that is, looking to the left in Fig. 3, the slot 64 will appear generally as a straight slot. The slot 64 permits medical instruments, such as stabilization devices 66, for example a skull clamp, retractors, clamps, supports, etc., also collectively referred to as "tools" herein, to be supported, selectively moved with respect to the distal end 30 of the support plate 28 to desired positions and locked or secured in place. In the illustrated embodiment, the slot 64 has a dovetail shape that matches a mating dovetail on the tool to be mounted and secured to the tool support 54. For example, the tool support 54 may receive one end 68 of a transitional element 70. The other end 72 of the transitional element 70 is rotatably coupled to a swivel adapter 74. The swivel adapter, in turn, is coupled to a skull clamp 76. The skull clamp 76 is normally manufactured from radiolucent materials, for example, as described in U.S. Patent No. 5,276,927 issued to the assignee of the present invention.

As shown in Fig. 3, the support plate 28 is often used in a generally horizontal position such that the top of the operating table 22 is generally in line with the support plate 28. However, numerous surgical procedures require that the support plate 28 be tilted or pivoted up or down with respect to the end 25 of the table 22. The tilting or pivoting of the support plate 28 is accomplished by the mechanism illustrated in Fig. 5. The attachment base 40 includes a pair of housings 86 connected to a lower surface 41 at a location near the ends of the attachment base 40 (Fig. 4). The attachment base 40 and housings 86 may be cast or made from aluminum. The support shafts 44 are rigidly connected at one end to respective cross-shafts 88 that are rotatably mounted within the lateral side walls 90 of the housings 86. The cross-shafts 88 extend through brass bushings (not shown) mounted in the lateral side walls 90 and function as pivot pin in a hinge. The support shafts 44 function as fixed hinge members, and the housings function as movable hinge members. A ratchet wheel 92 is fixed at the center of each of the cross-shafts 88, and each ratchet wheel has notches 94 between teeth 96. The support shafts 44, cross-shafts 88, and ratchet wheels 92 are normally made from stainless steel.

Pawls 98 are shaped to mate with and fit into the notches 94 of respective ratchet wheels 92. Each pawl 98 is mounted on the end of a release shaft 100 that extends through a bore 102 of a respective housing 86. With the pawls 98 in the position illustrated in Fig. 5, they function to securely support their respective housings 86 and the support plate 28 in a generally horizontal position. A spring 104 provides a bias to forcibly maintain the pawls 98 within the slots 94. The pawls 98 and release shafts 100 are normally made of stainless steel.

As shown in Fig. 4, a release shaft or bar 106, normally made of aluminum or stainless steel, extends between the shafts 100 and the housings 86. By pulling on the bar 106, the shafts 100 move to the right as viewed in Fig. 5; and the pawls 98 are pulled out of engagement with respective ratchet notches 94. Once the pawls 98 is disengaged from the notches 94, the support plate 28, attachment base 40, and housings 86 are freely rotatable relative to respective stationary ratchet wheels 92, cross-shafts 88 and support shafts 44. Thus, the support plate 28 may be pivoted with respect to an axis of rotation 108 in the generally clockwise or counter-clockwise direction until the support plate 28 is at its desired angular position as shown in phantom in Fig. 5. Normally, the support plate 28 may be pivoted approximately 60° above and below its illustrated horizontal position. When the bar 106 is released, the springs 104 push their respective pawls 98 into the closest ratchet notches 94, thereby securing the support plate with the desired angle or tilt.

In use, referring to Fig. 1, the scanning system 20 and operating table 22 are brought into a surgical suite. The scanning system 20 has a toroid shape scanning element 110 with a central opening 112 defining an enclosed or encircled scanning zone with which the portion of the patient to be scanned is axially aligned. The scanning element 110 further has the capability of rotating or tilting within its base 114 with respect to a diametric horizontal axis. The distal end 30 of the support plate 28 is narrowed so that it can extend into the opening 112 without interference. If necessary, the head section (not shown) of the table 22 is removed therefrom, and the radiolucent table extension 26 is mounted to the table by inserting the support bars 44 into mating bores 46 on the end surface 47 of the table 22. The patient 116 is then positioned on the table in a posture suitable for a surgical procedure. The length of the support plate 28 is sized such that the patient's upper torso and head are accessible for scanning and surgical procedures. The portion of the patient's anatomy on which the surgical procedure is to be performed may be stabilized by various clamps and restraining devices, for example, the skull clamp 76. Further, the support plate 28 or the scanning element 110 may be tilted so that the desired posture and/or scanning plane is achieved.

When the desired surgical posture is achieved, normally the patient will have already been scanned; and the surgical planning and procedure can be performed. Thereafter, a portion of the radiolucent table extension 26 is then moved into the opening 112, for a follow-up scan. The extent to which the extension 26 is moved into the opening 112 depends on what portion of the head or upper torso is to be scanned. The initial alignment of the table extension may be determined by visual inspection; and thereafter, a scan made to determine exactly whether and to what extent the table extension may be out of alignment. Alternatively, the scanner may be equipped with LED's or other sources of light providing beams of light with which the table extension can be aligned. In another embodiment, the table 22 may have an alignment tab 124 (Fig. 1) which is moved into an alignment slot 124 on the scanner 110. When the tab 124 is properly seated in the slot 126, the table is properly aligned with the scanner 110. The scanning process is executed by the scanning machine moving the scanning element 110 incrementally in an axial direction and with each increment, a scan is taken. Thereafter, the extension 26 and the patient are removed from within the scanning element 110, either by moving the scanning machine 20 or the operating table 22. The scan data is then used in association with the imaging systems 24 to plan the surgical procedure. The surgical procedure is then performed, and thereafter, the patient may be moved back into the scanning machine 20, and the scanning process repeated. The scanning and imaging system may be used to gauge the effectiveness of the surgical procedure; and if necessary, further procedures performed. The above process may be executed any number of times with the patient remaining in the desired position on the same patient support.

Thus, the above-described operating table and radiolucent table extension has a significant advantage of not only being able to support a patient during a scanning process, but also support the patient in the identical posture during a surgical procedure. The radiolucent table extension permits an operating table that is normally nonradiolucent and inappropriate for scanning purposes to be used with a scanning machine. Further, the table extension may be tilted to accommodate different desired surgical postures and is sized and shaped to readily fit within the opening of a scanning element, whether in a horizontal or tilted position. Further, not only does the table position permit successive scanning and operative procedures on the upper torso and head of a patient, but the radiolucent table extension 26 readily supports the patient in a prone, or supine position.

Referring to Fig. 6, an alternative embodiment of the support plate 118 has an distal end 120 that is curved to generally follow the profile of the headrest 32. Further a tool support 122 extends along the periphery of the support plate 118 to a location at which the width of the support plate 118 begins to flare outwardly toward the width of the fixed end 32. Other than its length, the construction and function of the tool support 122 is substantially identical to the tool support 54 described earlier.

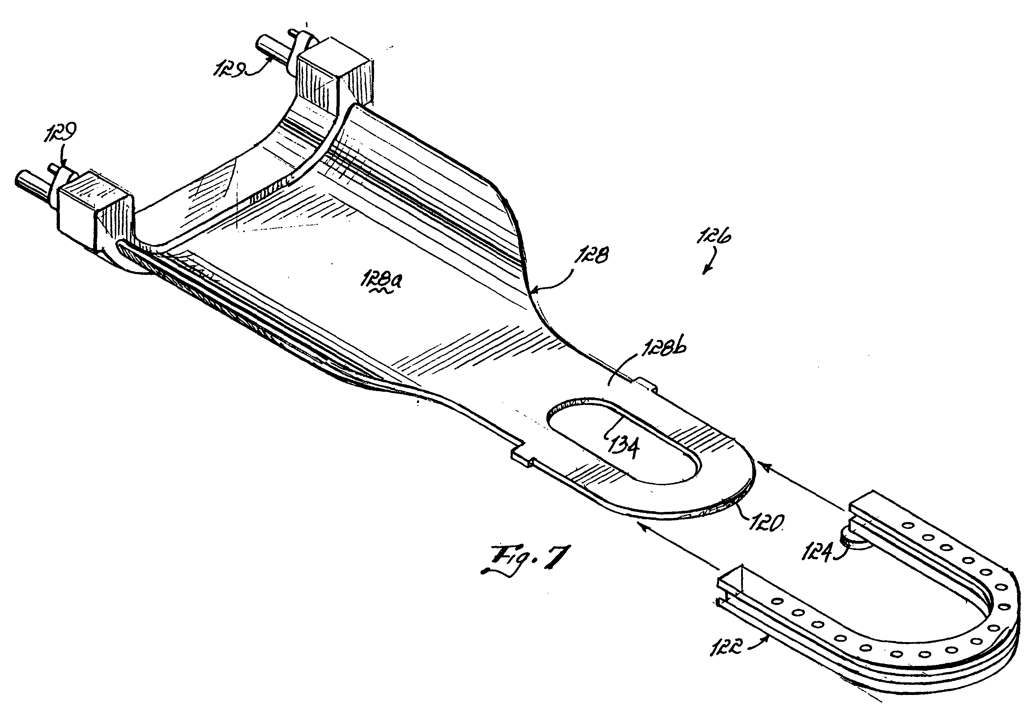

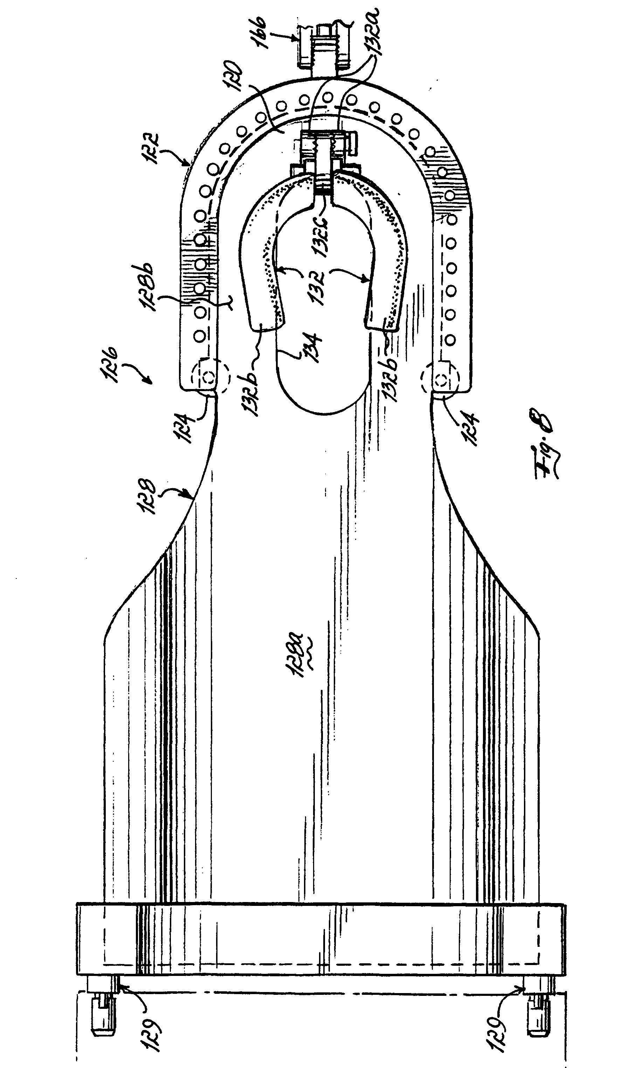

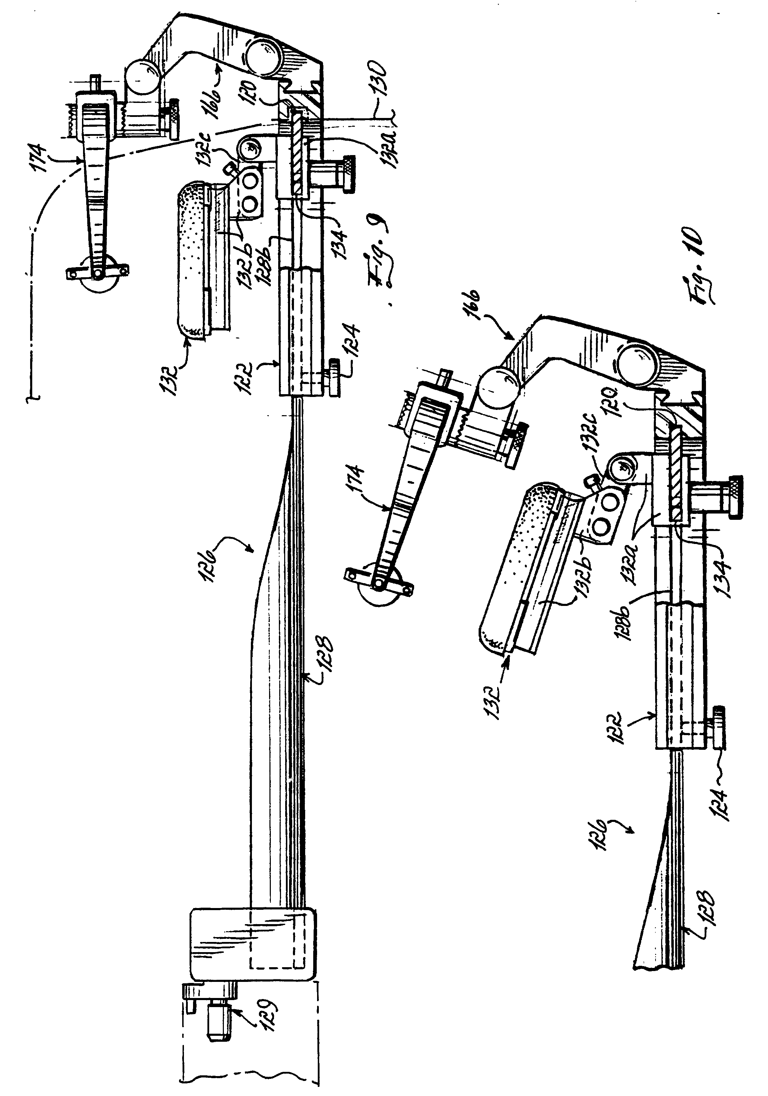

According to a further variation of this embodiment, as shown in Figs. 7-10, a radiolucent table extension assembly 126 includes a tool support 122 removably connected to the peripheral edge at a distal end 120 of the support member 128. The support member 128 is preferably pivotal relative to a table (not shown) to which is connected. This is done by incorporating a pivot mechanism (not shown) into the table extension assembly 126 or even into the table itself, as with surgical tables commercially available from Midmark of Dayton, Ohio, which are built so as to tilt relative to horizontal. With such tube, the support member 128 is simply plugged via pins 129 into the table (not shown) which is already oriented at a desired angle. The support member 128 may have an inner portion 128a which is contoured to the body of the patient and an outer portion 118b which is generally flat. The tool support 122 may removably secure to the support plate 128 via a pair of hand-tightenable knobs 124. The member 128 includes an opening 134, and a radiolucent horseshoe headrest 32 or 132 resides inboard of, and generally in alignment with, a portion of the opening 134 as shown in Figs. 8-10. This configuration enables a bag-like surgical drape (not shown) to be placed over a patient who is supported on the support member 128 by the horseshoe 132, and in an intubated condition, and then the tool support 122 connected to the distal end 120 to confine the drape within the edge of the support member 128, between the support member 128 and the tool support 122. Phantom line 130 in Fig. 9 illustrates an example of where this drape would be located. The patient 27 may be supported on the support member 128 in a face up or face down position. In a face down position, the hole 134 may be used for routing of one or more intubation tubes (not shown) or other medical instruments to the patient 27.

This results in locating the tool support 122 outside the drape 130, in the surgical field. This is also true for any other attendant hardware or assembly components 166 connected thereto, such as a skull clamp 174. For some types of surgical procedures, this draping arrangement may be preferable during surgical or scanning procedures. At least with respect to scanning, this configuration helps to assure that no structure will impede movement of the table extension assembly 126 into the scanning zone.

With this embodiment, i.e., the tool support 122 and the outer stabilization device 166, in this case the skull clamp 174, connected "outboard" of the outer edge of the support member 128, it is also possible to hold the head of the patient with a removably connected, tiltable horseshoe 132 located inboard of the edge of the support plate member 128 (Figs. 8, 9). Fig. 10 shows the headrest 132 tilted relative to the support member 128. With the tiltable horseshoe headrest 132, there is a first connection piece 132a which mounts to an inside edge of the opening 134 and a pair of mirror image headrest pieces 132b and 132c (Fig. 8) which connect to each other in a common plane and tilt relative to the piece 132a. This tilting feature gives the surgeon additional versatility in positioning the patient. Both the connector piece 132a and the second headrest pieces 132b and 132c are made of radiolucent material so as to not create artifacts during scanning.

Figs. 8-10 show outer tooling 166, specifically a skull clamp 174, along with an inner device such as a horseshoe headrest 132 connected to the tool support 122. Preferably the tooling or devices 166 are radiolucent and positively hold the patient in a fixed position relative to the support member 128, so that the patient remains in a desired position during successive surgery and scanning procedures. This is done with the inboard headrest 132 and/or an outer stabilization device 166, to affirmatively hold the patient 27 in a fixed position relative to the support plate 118. This structural capability facilitates convenient positioning of the patient 27 during successive scanning or surgical procedures, thereby enabling the surgeon to conveniently and easily perform follow-up procedures.

While the invention has been illustrated by the description of several embodiments and while the embodiments have been described in considerable detail, there is no intention to restrict nor in any way limit the scope of the appended claims to such detail. Additional advantages and modifications will readily appear to those who are skilled in the art. For example, the support plate 28 may be made of other radiolucent materials and may or may not have a laminated construction. Further, when viewed in a cross-section taken across its width as shown in Fig. 4, the support plate 28 has a curvature; however, the support plate 28 may also be constructed to be flat without such a curvature. Further, while the radiolucent table extension is particularly useful with nonradiolucent operating tables, it may also be used with radiolucent operating tables.

The tool support 54 has been described as an edge strip with a slot 64 having a dovetail shape for receiving and supporting tools; however, as will be appreciated, other configurations of the tool support are anticipated by the invention. For example, the slot 64 may have other shapes. Further, the slot 64 may be replaced by round, square or other shaped holes or coupling elements which are shaped to receive mating elements on tools, thereby supporting and holding the respective tools in a desired position. In addition, the tool support 54 may be a strip extending along the edge of the periphery of the plate 28 without the slot 64 but providing a hard surface for clamping purposes, for example, for using C-clamps to secure tools to the strip. While the tool support 54 is described and illustrated as having a slot 58 for receiving an edge of the plate 28, the tool support 54 can be attached to the plate 28 in other ways. For example, the slot 54 may be on the upper or lower surfaces of the plate 28, or the tool support 54 can be attached to the upper or lower surfaces, or the edge, of the plate 28. In addition, even though the tool support 54 has been described as being made from a radiolucent material, under some circumstances, for example, if the tool support is outside the scan field, the tool support 54 may be made of a nonradiolucent material, for example, metal.

As will be appreciated, the horseshoe-shaped gel filled headrest 32 illustrated and described may have other embodiments. For example, the headrest may be circular or another shape, may be filled with a different material, or may be thicker so that the patient's head is supported fully above the upper surface of the support plate 28. The headrest 132 shown in Figs. 8-10 represents only one of these possible variations. Further, the opening 34 may have other configurations. For example, the opening 34 may be replaced by, or supplemented by, one or a plurality of holes of any shape for various purposes, for example, ventilating the patient, access for tubes and other equipment, drainage, or openings through which the patient can see or the patient's eyes can be seen. As will be appreciated, separate inserts or built-in hole covers may be used to fill or cap the holes when they are not being used.

As will be appreciated, the aligning tab 124 may be located on the scanner 20, and the slot 126 located on the table 22. In addition, other alignment devices and procedures may be used. For example, the scanner 20 may have a built-in aligning system or the imaging system may be used to align the table 22 to the scanner 20.

## Claims

1. A table extension assembly (26, 126) for use in combination with a scanning machine (20) having an enclosed scanning zone, comprising:
a radiolucent member (28, 128) having a first end adapted to be attached to one end of a table (22) in cantilever fashion, the radiolucent member (28, 128) having a rigidity sufficient to support an upper torso and the head of a patient with the patient being further supported by an adjacently located surface of the table (22), the table (22) and the radiolucent member (28, 128) being movable relative to the scanning machine (20) to locate the member within the scanning zone,
**characterized by**
the member (28, 128) including an opening/hole (34, 134), and an inboard patient stabilization device (32, 132) for securely holding the head of the patient relative to the member (28, 128), which is in alignment with a portion of said opening/hole, and
a tool support (54, 122) extending along a periphery of the member (28, 128), the tool support (54, 122) being adapted to support at least one outboard patient stabilization device (66, 166) for stabilizing the head of the patient above the member (28, 128).

2. The table extension assembly of claim 1 wherein the table (22) has a first width and a distal end of the member (28, 128) has a width less than the first width of the table (22).

3. The table extension assembly of claims 1 or 2 and further comprising a base (40) connected to a first end of the member (28, 128) to removably mount the member (28, 128) to the table.

4. The table extension assembly of claim 3 and further including a mechanism (88-108) operatively connected to the base (40) or to the table (22) for permitting the member (28, 128) to be supported at a selected angle with respect to the table (22).

5. The table extension assembly of claim 4, wherein the mechanism (88-108) includes a locking element (100) for mechanically locking the member (28, 128) at the selected angle with respect to the table (22).

6. The table extension assembly of any of claims 1-5, wherein the means for locating includes an opening (34, 134) to assist in locating an inboard patient stabilization device for stabilizing the head of the patient.

7. The table extension assembly of any of claims 1-6, further comprising a radiolucent horseshoe headrest (32, 132) located near the means for locating and serving as an inboard patient stabilization device, the outboard stabilization device being a radiolucent skull clamp (76, 174).

8. The table extension assembly of claim 7 wherein the radiolucent horseshoe headrest (32, 132) has an inside perimeter and the member (28, 128) includes an opening (34, 134) residing within the perimeter of the headrest (32, 132), the opening (34, 134) is adapted to receive and encircle a portion of the head of the patient lying on the member (28, 128), thereby to stabilize the head of the patient relative to the member (28, 128).

9. The table extension assembly of any of claims 1-8 wherein at least a portion of the member (28, 128) has a contoured upper surface (128a) for conformingly supporting the upper torso of the patient.

10. The table extension assembly of any of claims 1-9 wherein the table is an operating table.

11. The table extension assembly of any of claims 1-10 wherein the tool support (54, 122) removably connects to the periphery of the member (28, 128) and includes at least one of the following for holding the at least one outboard stabilization device (66, 166) in a desired position:
a slot, and at least two holes of desired shape, thereby to hold the outboard stabilization device (66, 166) in the desired position.

12. The table extension assembly of claim 1 wherein the tool support (122) is U-shaped.

13. The table extension assembly of any of claims 7-12 wherein the inboard stabilization device is a radiolucent horseshoe headrest (32, 132) which is removably secured to the member (28, 128).

14. The table of claim 13 wherein the radiolucent horseshoe headrest (32, 132) is located above the extension and is tiltable with respect thereto.

15. The table extension assembly of any of claims 7-14 wherein the horseshoe headrest (32, 132) resides within a surgical drape (130) surrounding the member (28, 128) and the tool support (122) and the skull clamp (174) reside external to the drape (130).

16. A scanning arrangement comprising:
a scanning machine (20) having a toroidal shape defining a scanning zone and being adapted to take scans in the scanning zone;
an imaging system operatively connected to the scanning machine (20) and being adapted to store images representative of scans of the scanning zone taken by the scanning machine (20) ;
a patient table (22) having an upper support surface, the patient table (22) and upper support surface including a radiolucent table extension assembly (26, 126) with a radiolucent member (28, 128) being adapted to support a head and upper torso of a patient residing on the upper support surface, the radiolucent table extension assembly (26, 126) being sized to be received within the scanning zone, with the head and the upper torso of the patient supported on the radiolucent member (28, 128), the patient table (22) and the radiolucent table extension assembly (26, 126) being movable relative to the scanning machine (20) to located the radiolucent table extension assembly (26, 126) within the scanning zone,
**characterized by** the radiolucent table extension assembly (26, 126) including means for locating (34, 134) at least one radiolucent inner stabilization device (32, 132) inboard of an outer edge of the table extension assembly (26, 126) to stabilize the head of the patient, and
a radiolucent tool support (54, 122) being located along the edge of the table extension assembly (26, 126) and holding a radiolucent outer stabilization device (66, 166) being adapted to securely hold the head of the patient in a fixed position relative to the table extension assembly (26, 126), wherein the one or both inner and outer stabilization devices (32, 66; 132, 166) being adapted to assure the accuracy of scans taken of the patient by the scanning machine (20) and subsequently stored in the imaging system.

17. The invention of claim 16 wherein the means for locating includes an opening (34, 134).

18. The invention of claim 16 wherein the radiolucent outer stabilization device (66, 166) is a skull clamp (76, 174).

19. The invention of claim 17 further comprising a horseshoe headrest (32, 132) serving as an inner stabilization device, the horseshoe headrest (32, 132) located adjacent the opening (34, 134) .

20. The invention of claim 19 wherein the horseshoe headrest (32, 132) is tiltable relative to the member (28, 128).

## Patentansprüche

1. Tischverlängerungsanordnung (26, 126) zur Verwendung in Verbindung mit einer Abtastvorrichtung (20), die einen abgeschlossenen Abtastbereich aufweist, mit:
- einem strahlendurchlässigen Glied (28, 128) mit einem ersten Ende, das ausgelegt ist, an einem Ende eines Tischs (22) in freitragender Weise angebracht zu werden, wobei das strahlendurchlässige Glied (28, 128) eine Steifigkeit aufweist, die ausreicht, um einen Oberkörper und den Kopf eines Patienten abzustützen, wobei der Patient ferner durch eine benachbart angeordnete Fläche des Tischs (22) zusätzlich abgestützt ist, wobei der Tisch (22) und das strahlendurchlässige Glied (28, 128) relativ zu der Abtastvorrichtung (20) bewegbar sind, um das Glied in dem Abtastbereich anzuordnen,
**gekennzeichnet durch**
- das Glied (28, 128), das eine Öffnung/Loch (34, 134) und eine innenseitige Patientenstabilisierungsvorrichtung (32, 132) zum sicheren Halten des Kopfs des Patienten relativ zu dem Glied (28, 128) umfasst, das mit einem Teil der Öffnung/Loch ausgerichtet ist, und
- eine Werkzeughalterung (54, 122), die sich längs eines Außenumfangs des Glieds (28, 128) erstreckt, wobei die Werkzeughalterung (54, 122) ausgelegt ist, wenigstens eine außenseitige Patientenstabilisierungsvorrichtung (66, 166) zum Stabilisieren des Kopfs des Patienten oberhalb des Glieds (28, 128) zu halten.

2. Tischverlängerungsanordnung nach Anspruch 1, bei der der Tisch (22) eine erste Breite und ein distales Ende des Glieds (28, 128) eine Breite kleiner als die erste Breite des Tischs (22) aufweisen.

3. Tischverlängerungsanordnung nach Anspruch 1 oder 2, und ferner mit einer Basis (40), die mit einem ersten Ende des Glieds (28, 128) verbunden ist, um das Glied (28, 128) entfernbar an dem Tisch zu befestigen.

4. Tischverlängerungsanordnung nach Anspruch 3, und ferner mit einem Mechanismus (88-108), der mit der Basis (40) oder mit dem Tisch (22) verbunden ist, um es zu ermöglichen, das Glied (28, 128) unter einem ausgewählten Winkel relativ zu dem Tisch (22) abzustützen.

5. Tischverlängerungsanordnung nach Anspruch 4, bei der der Mechanismus (88-108) ein Verriegelungselement (100) aufweist, um das Glied (28, 128) unter dem gewählten Winkel relativ zu dem Tisch (22) mechanisch zu verriegeln.

6. Tischverlängerungsanordnung nach einem der Ansprüche 1 bis 5, bei der die Einrichtung zum Lokalisieren eine Öffnung (34, 134) aufweist, um ein Anordnen einer innenseitigen Patientenstabilisierungsvorrichtung zum Stabilisieren des Kopfs des Patienten zu unterstützen.

7. Tischverlängerungsanordnung nach einem der Ansprüche 1 bis 6, ferner mit einer strahlendurchlässigen hufeisenförmigen Kopfstütze (32, 132), die nahe der Einrichtung zum Anordnen angeordnet ist und als innenseitige Patientenstabilisierungsvorrichtung dient, wobei die außenseitige Stabilisierungsvorrichtung eine strahlendurchlässige Schädelklemme ( 76, 174) ist.

8. Tischverlängerungsanordnung nach Anspruch 7, bei der die strahlendurchlässige hufeisenförmige Kopfstütze (32, 132) einen inneren Rand aufweist und das Glied (28, 128) eine Öffnung (34, 134) umfasst, die innerhalb des Rands der Kopfstütze (32, 132) liegt, wobei die Öffnung (34, 134) ausgelegt ist, um einen Teil des Kopfs des auf dem Glied (28, 128) liegenden Patienten aufzunehmen und zu umfassen, um dadurch den Kopf des Patienten relativ zu dem Glied (28, 128) zu stabilisieren.

9. Tischverlängerungsanordnung nach einem der Ansprüche 1 bis 8, bei der wenigstens ein Teil des Glieds (28, 128) eine konturierte obere Fläche (128a) aufweist, um den Oberkörper des Patienten formschlüssig abzustützen.

10. Tischverlängerungsanordnung nach einem der Ansprüche 1 bis 9, bei der der Tisch ein Operationstisch ist.

11. Tischverlängerungsanordnung nach einem der Ansprüche 1 bis 10, bei der die Werkzeughalterung (54, 122) entfernbar mit dem Außenumfang des Glieds (28, 128) verbunden ist und wenigstens eines der Folgenden umfasst, um die wenigstens eine außenseitige Stabilisierungsvorrichtung (66, 166) in einer gewünschten Position zu halten:
ein Schlitz und wenigstens zwei Löcher gewünschter Form, um dadurch die außenseitige Stabilisierungsvorrichtung (66, 166) in der gewünschten Position zu halten.

12. Tischverlängerungsanordnung nach Anspruch 1, bei der die Werkzeughalterung (122) U-förmig ist.

13. Tischverlängerungsanordnung nach einem der Ansprüche 7 bis 12, bei der die innenseitige Stabilisierungsvorrichtung eine strahlendurchlässige hufeisenförmige Kopfstütze (32, 132) ist, die entfernbar an dem Glied (28, 128) gesichert ist.

14. Tischverlängerungsanordnung nach Anspruch 13, bei der die strahlendurchlässige hufeisenförmige Kopfstütze (32, 132) oberhalb der Verlängerung angeordnet und relativ zu dieser kippbar ist.

15. Tischverlängerungsanordnung nach einem der Ansprüche 7 bis 14, bei der die hufeisenförmige Kopfstütze (32, 132) innerhalb einer chirurgischen Abdeckung (130) liegt, die das Glied (28, 128) und die Werkzeughalterung (122) umgibt, und die Schädelklemme (174) außerhalb der Abdeckung (130) liegt.

16. Abtastanordnung mit:
- einer Abtastvorrichtung (20), die eine ringförmige, einen Abtastbereich definierende Form aufweist und ausgelegt ist, um in dem Abtastbereich Abtastungen aufzunehmen,
- einem Bildgebungssystem, das betriebsfähig mit der Abtastvorrichtung (20) verbunden und ausgelegt ist, um Bilder zu speichern, die von der Abtastvorrichtung (20) aufgenommene Abtastungen des Abtastbereichs wiedergeben,
- einem Patiententisch (22) mit einer oberen Auflagefläche, wobei der Patiententisch (22) und die obere Auflagefläche eine strahlendurchlässige Tischverlängerungsanordnung (26, 126) mit einem strahlendurchlässigen Glied (28, 128) aufweisen, das ausgelegt ist, einen Kopf und einen Oberkörper eines auf der oberen Auflagefläche liegenden Patienten abzustützen, wobei die strahlendurchlässige Tischverlängerungsanordnung (26, 126) zur Aufnahme in der Abtastzone dimensioniert ist, wobei der Kopf und der Oberkörper des Patienten auf dem strahlendurchlässigen Glied (28, 128) abgestützt sind, wobei der Patiententisch (22) und die strahlendurchlässige Tischverlängerungsanordnung (26, 126) relativ zu der Abtastvorrichtung (20) bewegbar ist, um die strahlendurchlässige Tischverlängerungsanordnung (26, 126) innerhalb des Abtastbereichs anzuordnen,
**gekennzeichnet durch**
die strahlendurchlässige Tischverlängerungsanordnung(26, 126), die eine Einrichtung zum Anordnen (34, 134) wenigstens einer strahlendurchlässigen inneren Stabilisierungsvorrichtung (32, 132) innerhalb eines Außenrandes der Tischverlängerungsanordnung (26, 126) aufweist, um den Kopf des Patienten zu stabilisieren, und
- eine strahlendurchlässige Werkzeughalterung (54, 122), die längs des Randes der Tischverlängerungsanordnung (26,126) angeordnet ist und eine strahlendurchlässige äußere Stabilisierungsvorrichtung (66, 166) hält, die ausgelegt ist, um den Kopf des Patienten sicher in einer fixierten Position relativ zu der Tischverlängerungsanordnung (26, 126) zu halten, wobei eine oder beide inneren und äußeren Stabilisierungsvorrichtungen (32, 66; 132, 166) ausgelegt sind, um die Genauigkeit von Abtastungen zu gewährleisten, die von dem Patienten von der Abtastvorrichtung (20) aufgenommen und nachfolgend in dem Bildgebungssystem gespeichert werden.

17. Abtastanordnung nach Anspruch 16, bei der die Einrichtung zum Anordnen eine Öffnung (34, 134) aufweist.

18. Abtastanordnung nach Anspruch 16, bei der die strahlendurchlässige äußere Stabilisierungsvorrichtung (66, 166) eine Schädelklemme (76, 174) ist.

19. Abtastanordnung nach Anspruch 17, ferner mit einer hufeisenförmigen Kopfstütze (32, 132), die als innere Stabilisierungsvorrichtung dient, wobei die hufeisenförmige Kopfstütze (32, 132) benachbart der Öffnung (34, 134) angeordnet ist.

20. Abtastanordnung nach Anspruch 19, bei der die hufeisenförmige Kopfstütze (32, 132) relativ zu dem Glied (28, 128) kippbar ist.

## Revendications

1. Un ensemble extension de table (26, 126) pour utilisation en combinaison avec un scanographe (20) ayant une zone de balayage incorporée, comprenant :
un élément radiotransparent (28, 128), présentant une première extrémité, adaptée pour être fixée à une extrémité d'une table (22) en porte-à-faux, l'élément radiotransparent (28, 128) ayant une rigidité suffisante pour supporter une partie supérieure du torse et la tête d'un patient, le patient étant en outre supporté par une surface adjacente de la table (22), la table (22) et l'élément radiotransparent (28, 128) étant déplaçables par rapport au scanographe (20) pour positionner l'élément à l'intérieur de la zone de balayage,
**caractérisé par le fait que**
l'élément (28, 128) comprend une ouverture/trou (34, 134) et un dispositif de stabilisation du patient en position intérieure (32, 132) pour maintenir de façon sûre la tête du patient par rapport à l'élément (28, 128), qui est en alignement avec une partie de ladite ouverture/trou, et
un support d'outils (54, 122) s'étendant le long d'une périphérie de l'élément (28, 128), le support d'outils (54, 122) étant adapté pour supporter au moins un dispositif de stabilisation du patient en position extérieure (66, 166), pour stabiliser la tête du patient au-dessus de l'élément (28, 128).

2. L'ensemble extension de table selon la revendication 1, dans lequel la table (22) présente une première largeur et une extrémité distale de l'élément (28, 128) présente une largeur inférieure à la première largeur de la table (22).

3. L'ensemble extension de table selon la revendication 1 ou 2, et comprenant en outre une base (40) reliée à une première extrémité de l'élément (28, 128) pour monter de façon amovible l'élément (28, 128) sur la table.

4. L'ensemble extension de table selon la revendication 3, et comprenant en outre un mécanisme (88 à 108) relié fonctionnellement à la base (40) ou à la table (22) pour permettre à l'élément (28, 128) d'être supporté à un angle sélectionné par rapport à la table (22).

5. L'ensemble extension de table selon la revendication 4, dans lequel le mécanisme (88 à 108) comprend un élément de verrouillage (100) pour verrouiller mécaniquement l'élément (28, 128) à la valeur angulaire sélectionné par rapport à la table (22).

6. L'ensemble extension de table selon l'une quelconque des revendications 1 à 5, dans lequel les moyens de positionnement comprennent une ouverture (34, 134) pour aider au positionnement d'un dispositif de stabilisation de patient en position intérieure, afin de stabiliser la tête du patient.

7. L'ensemble extension de table selon l'une quelconque des revendications 1 à 6, comprenant en outre un repose-tête (31, 32) radiotransparent en forme de fer à cheval placé à proximité des moyens de positionnement et servant de dispositif de stabilisation du patient en position intérieure, le dispositif de stabilisation en position extérieure étant un serre-crâne (76, 174) radiotransparent.

8. L'ensemble extension de table selon la revendication 7, dans lequel le repose-tête (32, 132) radiotransparent en forme de fer à cheval présente un périmètre intérieur, et l'élément (28, 128) comprend une ouverture (34, 134) résidant à l'intérieur du périmètre du repose-tête (32, 132), l'ouverture (34, 134) étant adaptée pour recevoir et encercler une partie de la tête du patient étendu sur l'élément (28, 128), de manière à stabiliser la tête du patient par rapport à l'élément (28, 128 ) .

9. L'ensemble extension de table selon l'une quelconque des revendications 1 à 8, dans lequel au moins une partie de l'élément (28, 128) présente une surface extérieure (128a) profilée, pour supporter, en s'adaptant à sa forme, la partie supérieure du torse du patient.

10. L'ensemble extension de table selon l'une quelconque des revendications 1 à 9, dans lequel la table est une table d'opération.

11. L'ensemble extension de table selon l'une quelconque des revendications 1 à 10, dans lequel le support d'outils (54, 122) est relié de façon amovible à la périphérie de l'élément (28, 128) et comprend au moins l'un des éléments suivants pour maintenir le au moins un dispositif de stabilisation en position extérieure (66, 166) dans une position souhaitée :
une fente, et au moins deux trous de forme souhaitée, de manière à maintenir le dispositif de stabilisation en position extérieure (66, 166) dans la position souhaitée.

12. L'ensemble extension de table selon la revendication 1, dans lequel le support d'outils (122) est en forme de U.

13. L'ensemble extension de table selon l'une quelconque des revendications 7 à 12, dans lequel le dispositif de stabilisation en position intérieure est un repose-tête (32, 132) radiotransparent en forme de fer à cheval qui est fixé de façon amovible sur l'élément (28, 128).

14. L'ensemble extension de table selon la revendication 13, dans lequel le repose-tête (32, 132) radiotransparent en forme de fer à cheval est placé au-dessus de l'extension et est inclinable par rapport à celle-ci.

15. L'ensemble extension de table selon l'une quelconque des revendications 7 à 14, dans lequel le repose-tête (32, 132) en forme de fer à cheval, est placé à l'intérieur d'un champ opératoire (130) chirurgical, entourant l'élément (28, 128), et le support d'outils (122) et le serre-crâne (127) sont situés à l'extérieur du champ opératoire (130).

16. Un montage de scanographe, comprenant :
un scanographe (20), ayant une forme toroïdale définissant une zone de balayage et étant adapté pour effectuer des balayages dans la zone de balayage ;
un système d'imagerie, relié fonctionnellement au scanographe (20) et étant adapté pour stocker des images représentatives des balayages de la zone de balayage, faites par le scanographe (20) ;
une table pour patient (22), ayant une surface support supérieure, la table pour patient (22) et la surface support supérieure comprenant un ensemble extension de table (26, 126) radiotransparent avec un élément (28, 128) radiotransparent étant adapté pour supporter une tête et la partie supérieure du torse d'un patient étendu sur la surface support supérieure, l'ensemble extension de table (26, 126) radiotransparent étant dimensionné pour être logé à l'intérieur de la zone de balayage, avec la tête et la partie supérieure du torse du patient supportées sur l'élément (28, 128) radiotransparent, la table pour patient (22) et l'ensemble extension de table (26, 126) radiotransparent étant déplaçables par rapport au scanographe (20), pour placer l'ensemble extension de table (26, 126) radiotransparent à l'intérieur de la zone de balayage,
**caractérisé par le fait que** l'ensemble extension de table (26, 126) radiotransparent comprend des moyens pour placer (24, 134) au moins un dispositif de stabilisation intérieur (32, 132) radiotransparent en position intérieure d'un bord extérieur de l'ensemble extension de table (26, 126), afin de stabiliser la tête du patient, et un support d'outils (54, 122) radiotransparent étant placé sur le bord de l'ensemble extension de table (26, 126) et maintenant un dispositif de stabilisation extérieur (66, 126) radiotransparent, étant adapté pour maintenir de façon sûre la tête du patient en une position fixe par rapport à l'ensemble extension de table (26, 126), dans lequel l'un ou les deux parmi les dispositifs de stabilisation intérieur et extérieur (32, 66 ; 132, 166) sont adaptés pour assurer la précision des balayages pris du patient, à l'aide du scanographe (20) et, ensuite, mémorisées dans le système d'imagerie.

17. L'invention selon la revendication 16, dans laquelle les moyens de positionnement comprennent une ouverture (34, 134).

18. L'invention selon la revendication 16, dans laquelle le dispositif de stabilisation extérieur (66, 166) radiotransparent est un serre-crâne (76, 174).

19. L'invention selon la revendication 17, comprenant en outre un repose-tête (32, 132) en forme de fer à cheval, servant de dispositif de stabilisation intérieur, le repose-tête (32, 132) en forme de fer à cheval étant placé de façon adjacente à l'ouverture (34, 134).

20. L'invention selon la revendication 19, dans laquelle le repose-tête (32, 132) en forme de fer à cheval est inclinable par rapport à l'élément (28, 128).
